(19) Europäisches Patentamt
European Patent Office
Office européen des brevets

(11) Publication number: **0 034 172**
**B1**

(12) # EUROPEAN PATENT SPECIFICATION

(45) Date of publication of patent specification: **18.05.83**

(51) Int. Cl.³: **C 07 C 53/128,**
A 01 N 37/00, A 61 K 31/19

(21) Application number: **80901740.3**

(22) Date of filing: **28.07.80**

(86) International application number:
**PCT/US80/00954**

(87) International publication number:
**WO 81/00562 05.03.81 Gazette 81/6**

(54) **MIXED SALT OF VALPROIC ACID.**

(30) Priority: **20.08.79 US 68284**

(43) Date of publication of application:
**26.08.81 Bulletin 81/34**

(45) Publication of the grant of the patent:
**18.05.83 Bulletin 83/20**

(84) Designated Contracting States:
**DE FR GB NL SE**

(56) References cited:
**FR - M - 2 442**
**US - A - 4 127 604**

**Chemical Abstracts, Volume 76, No. 15, issued 20 April 1972 (Columbus, Ohio, U.S.A.), K. Shutto et al, Pharmacological Studies on sodium dipropylacetate. Anticonvulsant Acitives and General Pharmacological Actions', page 20, column 1**

(73) Proprietor: **ABBOTT LABORATORIES**
**14th Street and Sheridan Road**
**North Chicago, Illinois 60064 (US)**

(72) Inventor: **Meade Edwin M.**
**888 Martin Place, R.R. 1 Stamps Road**
**Duncan, British Columbia (CA)**

(74) Representative: **Hayward, Denis Edward Peter et al,**
**Lloyd Wise, Tregear & Co. Norman House 105-109 Strand**
**London WC2R OAE (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

Mixed salt of valproic acid

This invention relates to mixed salts of valproic acid having anticonvulsant activity.

In the last decade, 2-propylpentanoic acid and its alkali or earth alkali salts (hereinafter referred to as valproic acid and valproates or valproate salts, respectively) have been introduced in the arsenal of drugs useful for treating epileptic seizures or convulsions. Most commonly used are valproic acid itself or its sodium salt. The former is a liquid and as such is less desirable for preparing an oral dosage form while the latter is a solid that has poor stability characteristics partially due to pronounced hygroscopicity.

In FR 2552M it was reported that dipropylacetic acid and certain of its inorganic and organic derivatives, including its alkali and earth alkali salts, have anticonvulsant as well as other pharmacological activities. Chemical Abstracts, Volume 76, 1972, p. 20 abstract No. 81125p also reports the anticonvulsant and other activities of sodium dipropylacetate.

It has now been found that a highly stable, non-hygroscopic, solid entity can be prepared from valproic acid and its salts, representing a single chemical molecule with well-defined physical characteristics, although a definite structure has not been assigned to this entity.

The new compound represents a single crystalline entity consisting of one molecule each of valproic acid or diethylacetic acid and a valproate salt, the cation of said salt being sodium or calcium. While it has not been determined for sure whether the new compound represents a solution of the valproic acid in the valproate salt or a complex between the two compounds, the new material has been tentatively assigned the following structure:

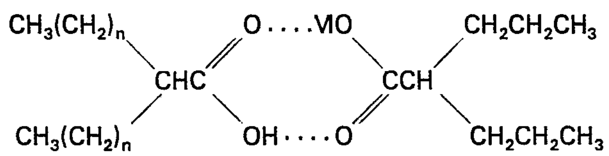

I

wherein M represents Na or 1/2 Ca and n is 1 or 2.

In the simplest embodiment, the above compound is prepared by dissolving one mole each of $[Me(CH_2)_n]_2$—CHCOOH and M-valproate in 1000 ml of acetone at about 50°C. After cooling the solution to 0°C. or below, the formed new compound is filtered, washed if desired with pre-cooled acetone, and dried under reduced pressure to remove all traces of acetone. Alternatively, the new compound wherein n=2 can be made in a two-component liquid medium which includes acetone. In this instance, M-valproate is formed *in situ* by adding MOH at a level of one half of a molecular equivalent of the valproic acid present, preferably as a solution in an acetone-miscible solvent for said M—OH, e.g., water. Another method consists in simply admixing an aqueous solution of sodium hydroxide of at least 10% concentration, preferably 35—50%, and valproic acid, said sodium hydroxide being used in an amount corresponding to 48—52% of the stoichiometric amount of said valproic acid, and removing the water from the reaction mixture by evaporation. A further modification comprises the replacement of the above acetone by sufficient acetonitrile to form a clear solution at 50°C or above. The new dimer can be recovered from the liquid phase by evaporating the solvent(s) and, if desired, the new dimer can be recrystallized, for instance from acetone/water, from acetonitrile or others, or the material may be spray-dried, lypholized or purified by chromatography.

The new dimer represents a single chemical molecule as can be determined by microanalysis, nmr spectrum, mixed melting point determination, IR spectrum and/or X-ray diffraction. The new compound does not have the aforementioned detrimental physical characteristics of either of the two starting materials; it is a crystalline, stable solid. Surprisingly, such a useful dimer can be made only from valproic acid and diethylacetic acid on one side of the molecule, with the sodium or calcium salt of valproic acid. When other valproate salts are used, i.e., the potassium, ammonium or magnesium salts, the resulting dimer, either does not crystallize, does not form or is highly unstable in the presence of any atmospheric moisture. Conversely, when other fatty acids are used to replace $[CH_3(CH_2)_n]_2CHCOOH$, including other branched fatty acids, similarly deficient products or product mixtures are obtained.

The process for making the compounds of this invention are best illustrated by reference to the following examples which, however, are not intended to limit the invention in any respect.

Example 1

In 1000 ml of acetone at about 50°C is dissolved 166 g of sodium valproate and 144 g of valproic acid. The solution is cooled to about 0°C., filtered and the crystalline precipitate is washed with pre-cooled acetone at about 0°C. The new compound is obtained in a yield of 90% of theory. Additional material can be obtained by using the acetone filtrate in a subsequent batch.

The new material is a stable white, crystalline powder which melts at 98—100°C. Its moisture stability is established by placing samples of the material for 45 minutes in a controlled environment at

room temperature and 80% relative humidity. No weight gain is observed, while under the same condition, the simple sodium salt of valproic acid gains between 17 and 24% in weight.

The infrared spectrum is consistent with proposed structure I and has the following characterizing absorption bands: strong bands at 2957, 2872, 2932, 1685, 1555 and 1370 $cm^{-1}$. The first two of these indicate the various methyl groups, the last two are due respectively to the anti-symmetric and symmetric O—C—O-stretching vibrations of the carboxyl salt. The remaining strong bands indicate the stretching vibrations of the various methylene groups and the C=O in the carboxylic acid group, while the weak, broad bands at 2450 and 1900 $cm^{-1}$ are due to intramolecularly bonded OH groups of the carboxylic acid.

Example 2

In a 500 ml flask containing 7.2 g of valproic acid is added a slurry of 0.926 g of fresh calcium hydroxide in 100 ml of water. After increasing the volume by the addition of water to 250 ml and warming the mixture on a steam bath to about 50°C, some undissolved residue remains which, however, dissolves upon cooling. The water is then evaporated under vacuum and the residue is dissolved in 200 ml of hot acetone. The solution is filtered, concentrated to about 100 ml and cooled in an ice bath to yield 5.4 g of fine, white, crystalline calcium dihydrogen valproate melting at 175°C. The nmr- and IR-spectra and microanalysis confirm the named single-molecule chemical identification and the material is stable to normal storage conditions.

Example 3

In the fashion of Example 1 but using sodium valproate with the molar equivalents of dibutylacetic acid or diethylacetic acid, respectively, the corresponding hydrogen sodium mixed salts of the assumed Structure I with n=3 or 1, respectively are obtained. In the instance of dibutylacetic acid, a very hygroscopic product is obtained which is very difficult to handle and therefore unsuitable for pharmaceutical dosage forms. The mixed salt obtained with diethylacetic acid is a white crystalline powder which is stable to ordinary storage conditions and essentially nohygroscopic.

Example 4

In a comparison of anticonvulsant activities of
A: valproic acid (stable, liquid)
B: sodium valproate (hygroscopic solid)
C: mixed dimer (stable solid) of Example 1

the oral $ED_{50}$ based on equimolar valproic acid equivalents are established by standard procedures. The results are as follows:

| | A | B | C |
|---|---|---|---|
| Audiogenic seizures (mice) | 154 | 141 | 81 mg/kg |
| Metrazol seizures (mice) | <800 | 282 | 178 mg/kg |
| Metrazol seizures (rats) | 355 | 415 | 362 mg/kg |

In a bioavailability study carried out with (A) and (C) above in various animal species, the peak blood plasma levels of oral, equimolar doses are determined according to standard procedures, 30 minutes after drug administration.

| | A | C |
|---|---|---|
| Mouse (200 mg/kg) | 133.7 | 207.4 mg/kg |
| Rat (200 mg/kg) | 84.1 | 63.0 mg/kg |
| Dog (25 mg/kg) | 65.2 | 73.6 mg/kg |
| Dog (25 mg/kg) AUC* | 82.3 | 95.0 hr.mcg/ml |

*Area under the curve value for 0—7 hours.

From the above examples it will be seen that the new material has equal or better physiological properties than either valproic acid or sodium valproate. Since the new dimer salt has far superior physical characteristics than either "monomer" from which it is made, it greatly facilitates the preparation of solid pharmaceutical dosage forms, and specific amounts can be weighed out and blended with starch and/or other binders to form a flowable powder which can be forwarded to standard tableting machines after granulation. Neither the hygroscopic sodium salt of valproic acid nor the liquid valproic acid itself can be processed in this fashion without special precautions or absorbents.

The new compounds can be tableted in accordance with Example XIII of U.S. 3,325,361 and analogous methods. In these procedures, one or more diluents and/or excipients are used, e.g., starch, talcum powder, lubricants, disintegrators, flavoring agents and coloring agents. These additives, of course, are the usual pharmaceutically acceptable carriers or diluents employed in routine fashion by tablet formulators.

While the above structure I is the most likely true two-dimensional view of the sodium/hydrogen divalproate or valproate-diethylacetate and seems to be confirmed by IR and nmr spectra, by molecular weight and microanalytic values, it is possible that the two molecules bind to one another in some other fashion. Thus, the new material should be characterized not by depicting a structural formula but by reference to a single compound of formula

$$(C_3H_7)_2CHCO_2Na/R_2CHCO_2H \quad \text{or} \quad [(Pr_2CHCO_2)(R_2CHCO_2)]Na,H$$

wherein each R is ethyl or propyl, or by reference to sodium/hydrogen divalproate or sodium/hydrogen dipropylacetate diethylacetate, or the corresponding dihydrogen/calcium tetravalproate or -bis(diethylacetate)divalproate.

## Claims

1. A dimer of the formula

$$(C_3H_7)_2CHCO_2M/R_2CHCO_2H$$

wherein each R represents ethyl or propyl, and M is Na or 1/2 Ca.

2. The dimer of claim 1 wherein M is sodium.
3. The dimer of claim 2 wherein R is propyl.
4. The dimer of claim 2 wherein R is ethyl.
5. The dimer of claim 1 wherein M is half of a calcium cation.
6. The dimer of claim 5 wherein R is propyl.
7. The dimer of claim 5 wherein R is ethyl.
8. An oral pharmaceutical dosage form for treating the symptoms of epileptic seizures or convulsions, containing as the active principle a compound of the formula

$$(C_3H_7)_2CHCO_2M/R_2CHCO_2H$$

wherein M represents one half of a calcium cation or sodium, R is propyl or ethyl, together with one or more pharmaceutically acceptable excipients or diluents.

9. The oral dosage form of claim 8 wherein M is sodium.
10. The oral dosage form of claim 9 wherein R is propyl.
11. The oral dosage form of claim 9 wherein R is ethyl.
12. The oral dosage form of claim 8 wherein M is half of a calcium ion.
13. The oral dosage form of claim 12 wherein R is ethyl.
14. The oral dosage form of claim 12 wherein R is propyl.

## Revendications

1. Dimère ayant pour formule:

$$(C_3H_7)_2CHCO_2M/R_2CHCO_2H$$

où R représente de l'éthyle ou du propyle et M est Na ou 1/2 Ca.

2. Dimère selon la revendication 1, caractérisé en ce que M est du sodium.
3. Dimère selon la revendication 2, caractérisé en ce que R est du propyle.
4. Dimère selon la revendication 2, caractérisé en ce que R est de l'éthyle.
5. Dimère selon la revendication 1, caractérisé en ce que M est la moitié d'un cation de calcium.
6. Dimère selon la revendication 5, caractérisé en ce que R est du propyle.
7. Dimère selon la revendication 5, caractérisé en ce que R est de l'éthyle.
8. Dose pharmaceutique orale pour le traitement de symptomes d'attaques ou de convulsions épileptiques, contenant comme principe actif un composé de formule:

$$(C_3H_7)_2CHCO_2M/R_2CHCO_2H$$

où M représente la moitié d'un cation de calcium ou de sodium, R est du propyle ou de l'éthyle, avec un ou plusieurs excipients ou diluants acceptables pharmaceutiquement.

9. Dose orale selon la revendication 8, caractérisée en ce que M est du sodium.
10. Dose orale selon la revendication 9, caractérisée en ce que R est du propyle.
11. Dose orale selon la revendication 9, caractérisée en ce que R est de l'éthyle.
12. Dose orale selon la revendication 8, caractérisée en ce que M est la moitié d'un ion de calcium.
13. Dose orale selon la revendication 12, caractérisée en ce que R est de l'éthyle.
14. Dose orale selon la revendication 12, caractérisée en ce que R est du propyle.

**Patentansprüche**

1. Medikament der Formel

$$(C_3H_7)CHCO_2M/R_2CHCO_2H$$

in welcher jedes R Äthyl oder Propyl bedeutet und MNa oder 1/2 Ca.

2. Medikament nach Anspruch 1, dadurch gekennzeichnet, daß M Natrium bedeutet.

3. Medikament nach Anspruch 2, dadurch gekennzeichnet, daß R Propyl bedeutet.

4. Medikament nach Anspruch 2, dadurch gekennzeichnet, daß R Äthyl bedeutet.

5. Medikament nach Anspruch 1, dadurch gekennzeichnet, daß M ein halbes Kalzium-Kation bedeutet.

6. Medikament nach Anspruch 5, dadurch gekennzeichnet, daß R Propyl bedeutet.

7. Medikament nach Anspruch 5, dadurch gekennzeichnet, daß R Äthyl bedeutet.

8. Orale pharmazeutische Dosierungsform zur Behandlung der Symptome von epileptischen Anfällen und Krämpfen, die als wirksamen Anteil eine Verbindung der Formel

$$(C_3H_7)_2CHCO_2M/R_2CHCO_2H$$

besitzt, wobei M in dieser Formel ein halbes Kalzium-Kation oder ein Natrium-Kation bedeutet, R entweder Propyl oder Äthyl bedeutet, zusammen mit einem oder mehreren pharmazeutisch akzeptablen Bindemitteln oder Verdünnungsmitteln.

9. Orale Dosierungsform nach Anspruch 8, dadurch gekennzeichnet, daß M Natrium bedeutet.

10. Orale Dosierungsform nach Anspruch 9, dadurch gekennzeichnet, daß R Propyl bedeutet.

11. Orale Dosierungsform nach Anspruch 9, dadurch gekennzeichnet, daß R Äthyl bedeutet.

12. Orale Dosierungsform nach Anspruch 8, dadurch gekennzeichnet, daß M ein halbes Kalzium-Ion bedeutet.

13. Orale Dosierungsform nach Anspruch 12, dadurch gekennzeichnet, daß R Äthyl bedeutet.

14. Orale Dosierungsform nach Anspruch 12, dadurch gekennzeichnet, daß R Propyl bedeutet.